# EUROPEAN PATENT SPECIFICATION

(11) **EP 1 946 104 B1**
(45) Date of publication and mention of the grant of the patent: **27.10.2010**
(21) Application number: 06790288.2
(22) Date of filing: 28.09.2006
(51) Int. Cl.: G01N 33/53, G01N 29/02, G01N 29/036, G01N 29/22, G01N 33/577

(54) **METHODS FOR MEASURING PRESENCE OR ABSENCE OF ANALYTES.**
METHODEN ZUR MESSUNG DER GEGENWART ODER ABWESENHEIT VON ANALYTEN.
MÉTHODES DE MESURE DE LA PRÉSENCE OU L'ABSENCE D'ANALYTES.

(30) Priority: 28.09.2005 AU 2005905353
(43) Date of publication of application: 23.07.2008
(73) Proprietor: Commonwealth Scientific and Industrial Research Organisation, Campbell, ACT 2601 (AU)
(72) Inventor: RAGUSE, Burkhard, Gordon, New South Wales 2072 (AU); WIECZOREK, Lech, Macquarie Park, New South Wales 2113 (AU)
(74) Representative: Westwood, Joanna
(86) International application number: PCT/AU2006/001417
(87) International publication number: WO 2007/035991

(56) References cited:
- US-A- 4 735 906
- US-A- 5 656 428
- US-A- 5 656 428
- XUAN M. ET AL.: 'Immunohistochemical co-localization of lymphatics and blood vessels in oral squamous cell carcinomas' JOURNAL OF ORAL PATHOLOGY AND MEDICINE vol. 34, 2005, pages 334 - 339, XP003010694
- YOON D.Y. ET AL.: 'Use of progesterone-3(O-carboxymethyl oxime)-horseradish peroxidase in a sensitive microtitre-plate EIA and its application to a visual membrane EIA of progesterone' JOURNAL OF IMMUNOASSAY vol. 16, no. 2, 1995, pages 137 - 153, XP008126004
- VALNES K. ET AL.: 'Paired indirect immunoenzyme staining with primary antibodies from the same species. Application of horseradish peroxidase and alkaline phosphatase as sequential labels' HISTOCHEMICAL JOURNAL vol. 16, 1984, pages 477 - 487, XP002977215

## Description

### FIELD OF THE INVENTION

The present invention relates broadly to the area of chemical and biochemical sensors, in particular the area of immunosensors and biosensors, devices comprising such sensors and methods of forming such sensors.

### BACKGROUND OF THE INVENTION

A known sensing technology is based on sandwich type assays for sensing an analyte in a sample, wherein a first receptor is bound to a substrate, the analyte binds to the receptor and a second receptor carrying a signal enhancing group is then allowed to bind to the analyte (see for instance Taussig et al Targets, (2003), 2(4), 169, Seong et al, Proteomics (2003), 3, 2176; Wu et al, Clinica Chimica Acta, 369, (2006), 119 and references therein). These sandwich assays may also be used in a competitive assay format wherein an analyte-mimic containing the signal enhancing group is already bound to the first receptor but is displaced by analyte contained within the test sample.

Generally, optical methods are used to as the readout mechanism for these sensors. This has the drawback that optical systems are relatively expensive as they require precision instruments. This is especially true for assays requiring high sensitivity. Thus methods are required to enhance the sensitivity of sandwich assays without requiring elaborate or costly sensing equipment.

### SUMMARY OF THE INVENTION

The present inventors have invented a novel sensor transduction method to detect the binding of a second receptor or the displacement of the analyte-mimic using the generation of an impulsive noise due to the interaction of a trigger element, that is attached to the second receptor or analyte-mimic, with a substrate on exposure to a triggering impulse such as bursts of electromagnetic radiation. This has the advantage of being detectable by simple, low-cost microphonic noise detection systems with enhanced sensitivity compared to some optical or other detection means.

The present inventors have further found that the use of trigger elements which generate an impulsive noise when triggered are capable of more general application in the field of analyte sensing than the sandwich assays described above.

Accordingly, the present invention provides a method for measuring the presence or absence of an analyte in a sample, as claimed.

### BRIEF DESCRIPTION OF THE FIGURES

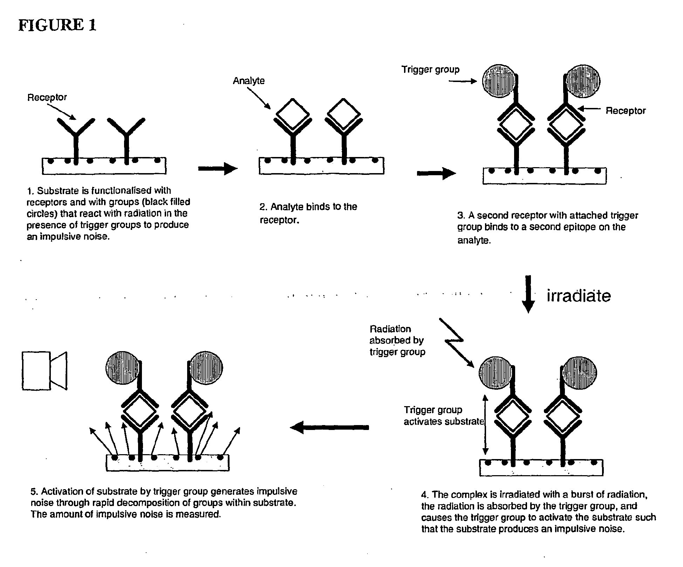

**Figure 1** shows a schematic diagram of a sandwich assay performed by means of the present invention. A first receptor is bound onto a reactive substrate. This is then exposed to a solution containing analyte and the analyte binds to the first receptor. A second receptor that has been functionalised with a trigger element (such as a nanoparticle) also binds to the analyte. The second receptor that has been functionalised with the trigger element may be present initially in a dehydrated form on the substrate or may be added as a second step after the analyte binds to the first receptor. A sandwich structure between the first receptor, the analyte, the second receptor onto which is conjugated the trigger element is thus formed on the reactive substrate. On exposure of the structure to a burst of irradiation, the trigger element absorbs at least part of the energy of the radiation, producing a localised hot spot, which in turn causes the substrate to at least partially decompose rapidly. The rapid decomposition reaction produces gaseous products that produce an impulsive noise. This impulsive noise is then measured by means of a microphone and amplifier system. The level of impulsive noise produced is related to the number of trigger elements near the substrate, which in turn is related to the number of analyte molecules in the test solution.

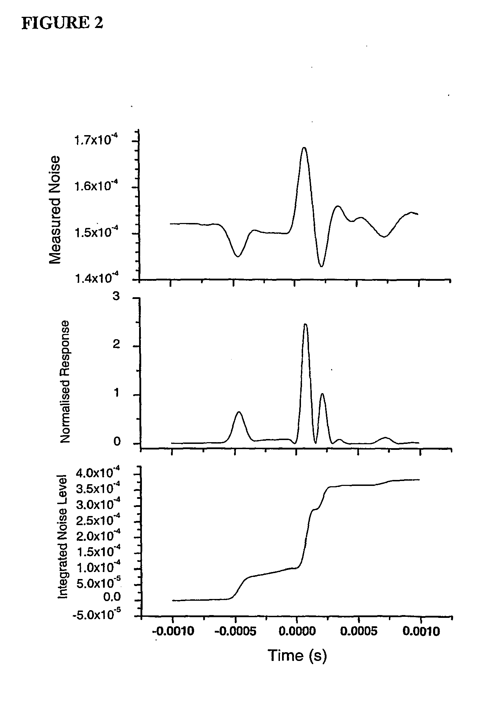

**Figure 2** shows a representative example of impulsive noise generated according to example 4. In this case figure 2a shows the impulsive noise measured by a microphone for 1x10¹⁰ Au16 nanoparticles absorbed onto a nitrocellulose membrane. Figure 2b shows the normalised and squared value of the impulsive noise that is integrated (figure 2c) in order to obtain the numerical value used for the present studies in example 3. It should be noted that numerous other methods of analysing the impulsive noise could be used effectively.

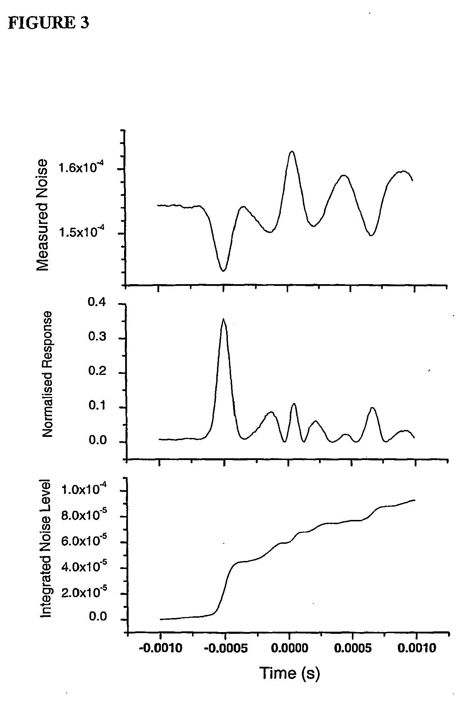

**Figure 3** shows a representative example of the impulsive noise generated according to example 4 in the case where no Au16 nanoparticles are absorbed onto the nitrocellulose membrane. The impulsive noise levels are significantly lower than for the case with nanoparticles adsorbed onto the nitrocellulose membrane.

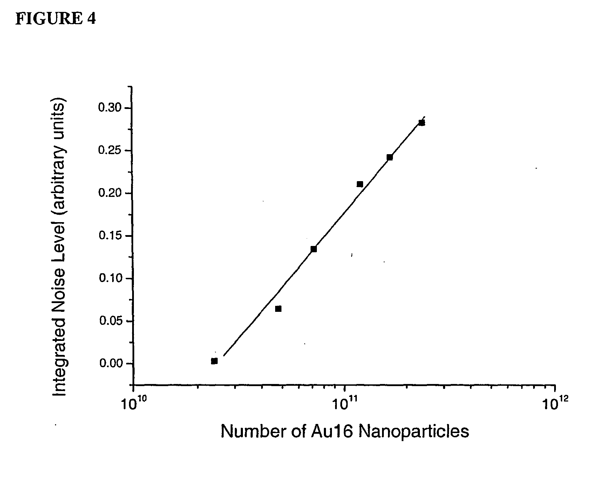

**Figure 4** shows the titration curve obtained in example 3 for different quantities of Au16 nanoparticles absorbed onto nitrocellulose membranes at relatively high nanoparticle loadings.

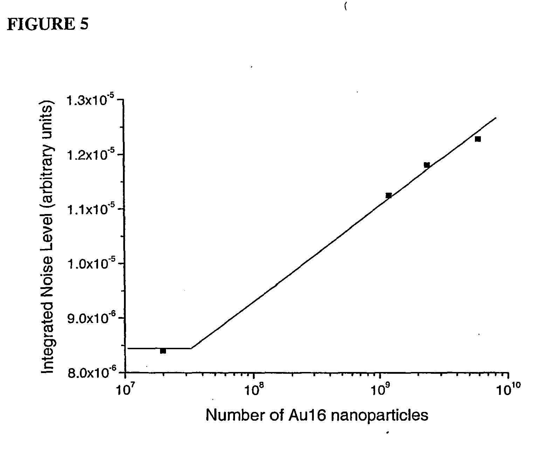

**Figure 5** shows an example of a titration curve obtained in example 3, for different quantities of Au16 nanoparticles adsorbed onto nitrocellulose membranes at very low nanoparticle loadings.

### DETAILED DESCRIPTION OF THE INVENTION

In a first aspect, the present invention provides a method for measuring the presence or absence of an analyte in a sample, wherein the presence or absence of the analyte is indicated by the respective presence or absence of one or more trigger elements that generate an impulsive noise when triggered, comprising the steps of:
(a) exposing the one or more trigger elements to a triggering impulse; and
(b) Detecting the impulsive noise generated by said one or more trigger elements, and wherein the trigger element interacts with the reactive substrate to generate the impulsive noise

As used herein, the term "analyte" means any substance for which there exists a naturally occurring specific binding member (receptor) or for which a specific binding member (receptor) can be prepared. The analyte may be for example proteins, peptides, antigens, bacterial antigens, glycolipids, viruses or viral fragments (hepatitis A, hepatitis B, hepatitis C, hepatitis delta, hepatitis E, malaria, tuberculosis, human immunodeficiency virus), haptens, antibodies (monoclonal or polyclonal), nucleic acid sequences, enzymes, hormones, vitamins, steroids, drugs (therapeutic or drugs of abuse), carbohydrates, explosive substances, toxins (ricin, nerve gas agents), pollutants (pesticides, endocrine hormone disrupting substances), cancer markers and so on.

The impulsive noise provides an indication of the presence of the analyte in the sample. Preferably, the impulsive noise provides an indication of the amount of analyte present in the sample. In a more preferred form, the intensity of the impulsive noise is directly related to the amount of analyte in the sample. The relationship between the intensity of the noise and the amount of analyte may be proportional or inversely proportional. For instance, in a competitive binding assay, if a greater the amount of analyte is present in the sample, then a lesser amount of trigger element is present and, accordingly, the intensity of the impulsive noise will be lower.

Preferably, the triggering impulse is radiation. Preferably, the radiation is selected from ultraviolet, visible, infra-red and microwave radiation and combinations thereof.

Preferably, the radiation is in the form of a pulse of less than 100 milliseconds duration but greater than one nanosecond duration. More preferably, the duration is less than 5 milliseconds.

The source of the radiation may be derived from a pulsed laser, a photoflash, a high power light emitting diode (LED), a pulsed xenon or a pulsed mercury lamp. More preferably, the source of the radiation is derived from a photoflash. The method for producing the pulse of radiation may be either through turning the radiation source on and off rapidly or through another mechanism such as a mechanical shutter device.

The triggering impulse need not be radiation. It can include other suitable means such as the use of an electrical current.

The trigger element can be any molecule or group that when exposed to specific conditions (ie the particular triggering impulse) is capable of generating an impulsive noise, either by itself or by interaction with a nearby reactive substrate.

Accordingly, in a preferred form, the trigger element interacts with a reactive substrate to generate the impulsive noise. The reactive substrate is preferably made from a material that in the absence of the trigger element is stable towards the triggering impulse but at least partly decomposes rapidly when the trigger element is exposed to the triggering impulse.

In a preferred embodiment, at least a proportion of the reactive substrate is a nitrated cellulose, nitrated starch, nitrated carbohydrate such as nitro-mannitol, 3-nitro-1,2,4-triazol-5-one, 2,4,6-trinitrobenzaldehyde, 2,4,6-trinitrotoluene, 2,4,6-trinitroanisole, 2,4,6-trinitro-m-cresol, N-methyl-N,2,4,6-tetranitroaniline, 2,4-dinitrotoluene, 2,4,6-trinitro-m-xylene, 1,3,6,8-tetranitronapthalene, dipentaerythritol hexanitrate, 2,2',4,4'6,6'-hexanitrobiphenyl, tetranitropentaerythritol, nonanitroterphenyl, octanitroterphenyl, ammonium-2,4,5-trinitroimidazole, hexahydro-1,3,5-trinitro-1,3,5-triazine, octahydro-1,3,5,7-tetranitro-1,2,5,7-tetrazine, 1,2,4-trinitrobenzene, 1,3,5-trinitrobenzene, 1,3-diamino-2,4,6-trinitrobenzene.

It is preferred that the reactive substrate comprises an oxidising polymer produced from a polymer substance that is partially nitrated, that is, an organic polymer functionalised with organo-nitro groups.

It is further preferred that the reactive substrate contains nitrated cellulose, nitrated starch, nitrated carbohydrate such as nitro-mannitol.

In a preferred example, at least a proportion of the reactive substrate is an inert material. An inert material, in the context of the present invention, is a material that does not generate an impulsive noise during or after exposure to a triggering impulse in either the presence or absence of the trigger element.

It is further preferred that the reactive substrate comprises an inert material that is at least partially coated with a thin film of a substance that does produce an impulsive noise on exposure to a triggering impulse in the presence of the trigger group.

It is further preferred that the reactive substrate comprises a nitrated cellulose material such as nitrocellulose used for filtration membranes.

It is further preferred that the reactive substrate comprises a nitrocellulose material containing a proportion of cellulose acetate.

The proportion of cellulose acetate is preferably between 5 to 20 % w/w relative to the nitrocellulose.

It is further preferred that the reactive substrate is in the form of a porous substrate.

The reactive substrate is preferably located in close proximity to the trigger element. For instance, it may form a shell encapsulating the trigger element. Conversely, the substrate may be in the form of a particle with a diameter of between 1 to 10,000 nm wherein said particle is at least partly functionalised with the trigger element. In another preferred form, the substrate forms part of the region in which the sample is analysed. For instance, the surface of the region may comprise the substrate.

In a preferred form, the trigger element is a coloured, insoluble substance or precipitate. The present inventors have found that certain coloured substances or precipitates, such as those produced by enzymes interacting with the enzyme substrates can function as trigger elements. Enzymes capable of producing such coloured insoluble substances are well known to those skilled in the art and include peroxidases, phosphatases, esterases, oxidases such as horseradish peroxidase, alkaline phosphatase, acetylcholinesterase, choline oxidase. These enzymes catalyse the precipitation of a chromogenic substrate in the immediate vicinity of the enzyme. Chromogens may include nitro blue tetrazolium (NBT/BCIP), 3,3'-diaminobenzidine tetrahydrochloride (DAB), and 3-amino-9-ethylcarbazole (AEC).

Preferably the coloured, insoluble substance is produced by a peroxidase reacting with 3,3'diaminobenzidine tetrahydrochloride to produce a red-brown precipitate.

Preferably the coloured, insoluble substance is produced by a peroxidase reacting with 3,3'diaminobenzidine tetrahydrochloride in the presence of nickel ions to produce a blue-grey precipitate.

In a further preferred example an enzyme can be used to produce a metallic deposit on the reactive substrate, said metallic deposit acting as the trigger element. Such enzyme-catalysed metal deposition is known to those skilled in the art and is described in US2005/0100976 A1, which is incorporated by reference in its entirety.

In a preferred form, the trigger element is a nanoparticle. The present inventors have found that nanoparticles when exposed to specific conditions (ie particular triggering impulses) are capable of generating an impulsive noise from nearby reactive substrates.

Preferably, the nanoparticle is between 1 and 100 nm in diameter.

Preferably, the nanoparticle is made from metals such as gold, silver, palladium, copper, platinum, nickel, cobalt, chromium, iron, ruthenium, rhodium, or alloys of such metals; metal oxides such as titanium oxide, iron oxides, zinc oxides, nickel oxides, copper oxides, cobalt oxides and indium oxide; metal sulfides; metal selenides; carbon nanoparticles, carbon black, carbon nanotubes, carbon fullerenes; magnetic nanoparticles; clusters of dye molecules; or core-shell nanoparticles wherein a nanoparticle core is coated with another material to form a shell.

In the case where a nano-particle is a core-shell nanoparticle, the core-shell nanoparticle may be particles with a reactive metal core and an inert shell such as aluminium core-aluminium oxide shell nanoparticles.

The nanoparticles may be spherical, oblate spheroids, platelets, cubic, rod-like, hemi-spherical or other shape, depending on the desired absorption characteristics. It is known in the art that depending on the shape of the nanoparticle it is possible to change its absorption characteristics, thus rod-like gold nanoparticles absorb at longer wavelengths than spherical nanoparticles and hollow hemispherical gold nanoparticles may have significant absorption characteristics in the near infra-red spectrum.

In one embodiment, the trigger element comprises a gold nanoparticle of 5 to 60 nm diameter.

In another example, the trigger element comprises a carbon black nanoparticle.

Nanoparticles are able to absorb part of the burst of radiation, giving rise to a high energy state where the nanoparticles specifically interacts with a reactive substrate such that it causes the substrate to produce an impulsive noise through rapid chemical degradation reactions.

Although not wishing to be bound by scientific theory, it is believed that the nanoparticle when in its high energy state after absorption of radiant energy, is able to catalyse the localised chemical breakdown of the reactive substrate, giving rise to a number of gaseous products. The rapid nature of this chemical reaction gives rise to pressure waves in the surrounding medium that manifest themselves as impulsive noise. This impulsive noise may be detected and analysed using microphone systems or other methods that are sensitive towards changes in pressure in gas, liquid or solid media.

The sensitivity of the method may be enhanced by techniques known in the art. For instance, in circumstances where the trigger element is a gold nanoparticle, the amount of nanoparticles present may be increased by the addition of an LI silver enhancer solution. The LI silver enhancer solution consists of a two-part electroless plating solution that autocatalytically plates silver onto gold nanoparticles, thereby increasing the size and optical density of the nanoparticles.

Accordingly, there is further provided a method according to the first aspect, said method comprising, before steps (a) and (b), the step of:
exposing said one or more trigger elements to a solution of electroless metal plating solution,
wherein said electroless metal plating solution deposits metal onto the triggering elements.

The means for detecting impulsive noise is preferably a microphone, a piezoelectric system or other means of detecting pressure changes in gas, solid or liquid media.

In one embodiment, the present invention provides a method for measuring the presence of an analyte in a sample, said method comprising, before steps (a) and (b), the steps of:
(i) immobilising first receptors in a region wherein the first receptors are capable of binding to the analyte;
(ii) adding the sample to the region whereby, when analyte is present in the sample, the analyte binds to said first receptors;
(iii) adding second receptors to the region wherein the second receptors bind to the analyte and wherein the second receptors comprise said one or more trigger elements; and
(iv) removing unbound second receptors from said region.

Steps (ii) and (iv) may be carried out in either order or simultaneously.

For example, the second receptors may be present to the region, perhaps in a dehydrated form, before the addition of the sample. Alternatively, the second receptors may be added to the region simultaneously with or after the addition of the sample.

In another embodiment, the present invention provides a method for measuring the presence of an analyte in a sample, said method comprising, before steps (a) and (b), the steps of:
(i) immobilising first receptors in a region wherein the first receptors are capable of binding to the analyte;
(ii) adding analyte-mimics to the region wherein the analyte-mimics bind to the first receptors and wherein the analyte-mimics comprise a trigger element that generates an impulsive noise when triggered;
(iii) adding the sample to the region whereby, when analyte is present in the sample, the analyte binds to said first receptors and displaces said analyte-mimics; and
(iv) removing unbound analyte-mimics from said region.

Steps (ii) and (iv) may be carried out in either order or simultaneously.

The analyte-mimics may be present in the region and bound to the first receptors before addition of the sample. Alternatively, the analyte-mimics may be added to the region simultaneously with or after the addition of the sample.

In the context of the present invention it is preferred that the receptors are antibodies such as monoclonal or polyclonal antibodies or their fragments capable of binding to an analyte, such as the Fab or Fv fragments, streptavidin, avidin, biotin, protein A, protein A and its complex with antibodies, proteins (natural or recombinant), protein scaffolds, protein G, protein G and its complex with antibodies, lectins, DNA, RNA, nucleic acid aptamers, enzymes, G-protein coupled receptors, G-proteins, anticalins, trinectins, affibody molecules, receptors based on molecularly imprinted polymers.

It is particularly preferred that the receptors are antibodies such as monoclonal or polyclonal antibodies or their fragments capable of binding to an analyte, such as the Fab or Fv fragments.

The analyte-mimics of the present invention are preferably functionalized analyte or hapten molecules that have been modified in order to attach them to the trigger group in such a way that they are capable of binding to the first receptors. A requirement of the analyte-mimic is that the first receptor/analyte-mimic binding properties are such that the analyte-mimic is able to replaced by the analyte of interest.

It is preferred that the first receptors are immobilised either by physical adsorption or via specific chemical interaction. Similarly, it is preferred that the second receptors and analyte-mimics are attached to the trigger element either by physical adsorption or via specific chemical interaction.

In the case of the trigger element being a metal nanoparticle such as a gold, platinum, palladium or silver nanoparticle, it is preferred that the receptor/analyte-mimic is adsorbed onto the metal surfaces via non-specific binding, or that specific chemical coupling strategies such as those known to those skilled in this art are used. Such specific coupling chemistries include, but are not limited to: using metal-sulfur interactions where the receptor/analyte-mimic contains a surface accessible thiol, disulfide, sulphide, or other sulfur moiety known to bind to gold, palladium, platinum or silver.

In a further preferred example, the trigger element that is attached to the receptor/analyte-mimic is further functionalised such that non-specific binding of other moieties in the sample to be analysed is minimised. This may be achieved through adsorbing proteins such as bovine serum albumin, or casein to the trigger group after functionalising with the receptor. It is also possible to minimise non-specific binding to the trigger element by functionalising the trigger element with compounds containing oligoethylene glycol or polyethylene glycol groups.

In a yet further embodiment, the present invention provides a method of measuring the presence of an analyte in a sample, said method comprising, before steps (a) and (b), the step of:
(i) exposing said sample to an analyte-specific substrate, wherein said analyte-specific substrate generate said one or more trigger elements in the presence of analyte.

Preferably the analyte-specific substrate is an enzyme capable of producing one or more trigger elements in the presence of analyte and include peroxidases, phosphatases, esterases, oxidases such as horseradish peroxidase, alkaline phosphatase, acetylcholinesterase, choline oxidase, glucose oxidase.

In a yet further embodiment, the present invention provides a method of measuring the presence of an analyte in a sample, said method comprising, before steps (a) and (b), the steps of:
(i) exposing said sample to an analyte-specific substrate, wherein said analyte-specific substrate adsorbs or binds to said analyte and wherein said analyte-specific substrate comprises one or more trigger elements; and
(ii) removing unadsorbed or unbound analyte-specific substrate.

Preferably, the analyte is a protein and the analyte-specific substrate is a nanoparticle, such as a gold nanoparticle. The present inventors have found that a sample of protein will adsorb gold nanoparticles in proportion to the amount of protein present.

It is further preferred that the analyte is a protein or mixture of proteins that have been subjected to a Western blot analysis wherein a sample containing one or more proteins is subjected to gel electrophoresis, followed by transfer of the separated proteins onto a nitrocellulose membrane. Protein blotting procedures, such as Western blotting techniques, are well known to those of ordinary skill in the art. A review of protein blotting is given in "Protein blotting: a review" by B.J. Kurien, R.H Scoffield; J. Immunol., Methods, 274 (2003) pg 1-15, which is incorporated by reference in its entirety.

It is further preferred that the present embodiment is a Southern (DNA) or Northern (RNA) blotting analysis.

The present disclosure further provides reagents, such as receptors and analyte-mimics, that can be used in the method of the present invention.

Accordingly, the present disclosure provides a receptor capable of binding to an analyte wherein the receptor is labelled with a trigger element wherein said trigger element generates an impulsive noise when triggered.

Further, the present disclosure provides an analyte-mimic capable of binding to a receptor for an analyte, wherein said analyte-mimic is labelled with a trigger element wherein said trigger element generates an impulsive noise when triggered.

### Example 1. Synthesis of nanoparticles

Standard synthetic methods were used for the synthesis of nanoparticles. Typical methods may be found in publications such as: Clusters and Colloids, from Theory to Applications, G. Schmid (Ed.), 1994, VCH Publishers New York, USA; Nanoparticles and Nanostructured Films, J.H. Fendler (Ed.), 1998, Wiely-VCH. Unless otherwise specified water-soluble, sodium citrate coated gold nanoparticles of approximately 16 nm in diameter (hereafter known as Au16 gold nanoparticles) were synthesised according to the method of Schmitt, J. *et. al. Adv. Mater.,* 1997, *9*, 61. The final concentration of Au16 nanoparticles was 2 nM with respect to the number of particles.

### Example 2. Synthesis Au16 gold nanoparticle conjugated with biotin

A sample solution of Au16 gold nanoparticles (20 ml) was treated with N-[6-(biotinamido)hexyl]-3'-(2'-pyridyldithio)propionamide (1ul of 10 mM, in dimethylsulfoxide). The Au16 gold nanoparticles were left for 30 minutes, resulting in functionalization of the Au16 nanoparticles with biotin (solution Au16-Biotin). The Au16-Biotin solution was stable for several days at 4°C and was used as a test analyte solution wherein the biotin represent the analyte to be determined conjugated to a Au16 gold nanoparticle as the trigger group.

### Example 3. Impulsive noise generation and detection by Au16 gold nanoparticles on nitrocellulose membrane substrates.

A solution of Au16 gold nanoparticles (10 ml) was treated with cystamine hydrochloride (100 µl, 0.1M aqueous solution). Rapid cross-linking of the nanoparticles occurred as evidenced by the rapid colour change from burgundy-red to blue-black. Aliquots of this solution were diluted to 1 ml with water and were then filtered through Millipore^{™} GSWP04700 membranes. These membranes are derived from nitrocellulose and have a nominal pore size of 0.22 um. The filter area used was approximately 0.28 cm². After filtration the cross-linked nanoparticles formed a thin film on and in the pores of the membrane. The membrane was dried and placed adjacent to a photoflash. The photoflash used was a Canon 550 unit operating at between one-eighth to half power. Other photoflash units from different manufacturers were also trialled and could be readily used. A microphone was placed adjacent to the membrane and connected to a digitally controlled potentiostat and data acquisition system (AdInstruments, Powerlab Data Acquisition System and Potentiostat). A voltage was applied to the microphone (0.5V) and changes in current was monitored as the microphone responded to different noise levels. The photoflash was then triggered, producing a brief burst of irradiation and the impulsive noise generated by the substrate with the absorbed Au16 nanoparticles was monitored. The resulting signal was normalised to a preflash initial current value of zero microamps, squared and integrated in order to obtain a value (hereafter called the integrated noise level) for the impulsive noise level generated. Figure 2a shows a typical noise response measured, figure 2b shows the normalised, squared signal and figure 2c shows the integrated response. Figure 3 shows a representative example of the impulsive noise generated according to example 4 in the case where no Au16 nanoparticles are absorbed onto the nitrocellulose membrane. The impulsive noise levels are significantly lower than for the case with nanoparticles adsorbed onto the nitrocellulose membrane.

Figure 4 shows a graph of the integrated noise level versus the varying number of nanoparticles in 1 ml of solution deposited onto a nitrocellulose filter membrane. The noise level was integrated over 25 milliseconds. Figure 5 shows a graph of the integrated noise level versus the varying number of nanoparticles in 1 ml solution deposited onto a nitrocellulose filter membrane, where only the noise peak occurring at time = 0 to 0.2 msec is measured.

### Example 4. Demonstration of sensing method - detection of biotin conjugated to a Au16 gold nanoparticle trigger group.

A Millipore^{™} GSWP04700 nitrocellulose membrane (0.28 cm²) was functionalised with a protein receptor, streptavidin (10 ul of 1 mg/ml) for 3 min. During this time the streptavidin non-specifically bound to the nitrocellulose membrane surface. The membrane was then placed onto a porous adsorbent membrane pad that acts as a reservoir for adsorbing the liquid from the applied sample. The membrane composite was then placed in a holder, forming a well above the membrane. A solution of Au16-Biotin (1 ml) was then placed in the well and allowed to flow into the adsorbent pad below the membrane by capillary forces. The Au16-Biotin nanoparticle analyte was bound to the streptavidin on the surface during this phase. The membrane was subsequently removed, dried and placed onto the photoflash and a microphone connected as in example 3. The integrated noise level was 0.16. A control experiment wherein the Millipore^{™} GSWP04700 nitrocellulose membrane was not firstly functionalised with streptavidin was also carried out in analogous manner. The integrated noise level was 0.01 clearly indicating that detection of the biotin analyte requires the streptavidin receptor bound onto the substrate.

### Example 5. Comparison of impulsive noise generation by Au16 gold nanoparticles on nitrocellulose membrane substrates of different membrane areas.

A solution of Au16 gold nanoparticles (10 ml) was treated with cystamine hydrochloride (100 ul, 0.1M aqueous solution). Rapid cross-linking of the nanoparticles occurred as evidenced by the rapid colour change from burgundy-red to blue-black. Aliquots of this solution were diluted with water as required and were then filtered through Millipore^{™} GSWP04700 membranes. These membranes are derived from nitrocellulose and have a nominal pore size of 0.22 um. Two samples of each nanoparticle aliquot were prepared and were filtered through membranes with two different areas. For sample A, the filter area used was approximately 0.28 cm², for sample B the filter area was approximately 0.027 cm². After filtration the cross-linked nanoparticles formed a thin film on and in the pores of the membrane. The membrane was dried and placed adjacent to a photoflash. The photoflash used was a Canon EX-550 unit operating at full power. A microphone system (Optima N26) was placed adjacent to the membrane and connected to a digitally controlled potentiostat and data acquisition system (AdInstruments, Powerlab Data Acquisition System and Potentiostat) using the preamplifier of the microphone system. The microphone system produced a current that was measured by the Powerlab Data Acquisition System and Potentiostat system. The absolute integral of the output signal was determined over the first four milliseconds after the photoflash was activated and for convenience was multiplied by a factor of 10⁶. This value of the absolute integral will hereafter be known as the integrated noise signal (INS). The results are shown in table 1 below.

**TABLE 1**

| Number of nanoparticles filtered onto membrane | INS for filter area of 0.28 cm² | INS for filter area of 0.027 cm² |
|---|---|---|
| 6 x 10⁸ | 2.2 | 2.4 |
| 1.2 x 10⁹ | 2.4 | 4.1 |
| 2.4 x 10⁹ | 2.5 | 12.6 |
| 6 x 10⁹ | 7.0 | 31.9 |
| 1.2 x 10¹⁰ | 125.3 | 40.8 |
| 2.4 x 10¹⁰ | 191.1 | 42.4 |
| 6 x 10¹⁰ | 248.1 | 38.5 |
| 1.2 x 10¹¹ | 276.7 | 44.6 |

As can be seen the size and shape of the nanoparticle film influences the amount of the INS. For instance, by decreasing the area of the filter an effective increase in the sensitivity of the detection limit of the number of nanoparticles may be achieved (i.e. an effective lower detection limit of 1.2 x 10⁹ nanoparticles for the 0.027 cm² area film compared to 6 x 10⁹ nanoparticles for the 0.28 cm² film). Alternatively by increasing the area it is possible to distinguish higher concentrations of nanoparticles effectively.

### Example 6. Comparison of impulsive noise generation by Au16 gold nanoparticles on nitrocellulose membrane substrates and an inert PVDF membrane

A solution of Au16 gold nanoparticles (10 ml) was treated with cystamine hydrochloride (100 ul, 0.1M aqueous solution). Rapid cross-linking of the nanoparticles occurred as evidenced by the rapid colour change from burgundy-red to blue-black. Aliquots of this solution were diluted with water as required and were then filtered through Millipor^{™} GSWP04700 membranes or Millipore^{™} Durapore GVWP04700membranes. These membranes are derived from nitrocellulose and a polyvinylidine fluoride (PVDF) polymer respectively and have a nominal pore size of 0.22 um. The filter area used was approximately 0.28 cm². After filtration the cross-linked nanoparticles formed a thin film on and in the pores of the membrane. The membrane was dried and placed adjacent to a photoflash. The INS was then obtained as for example 5. Results of the INS for the two types of membrane are shown in table 2 below.

**TABLE 2**

| Number of nanoparticles filtered onto membrane | INS for Nitrocellulose Membrane | INS for PVDF membrane |
|---|---|---|
| 6 x 10⁸ | 2.2 | 2.2 |
| 1.2 x 10⁹ | 2.4 | 2.3 |
| 2.4 x 10⁹ | 2.5 | 2.2 |
| 6 x 10⁹ | 7.0 | 2.5 |
| 1.2 x 10¹⁰ | 125.3 | 3.1 |
| 2.4 x 10¹⁰ | 191.1 | 3.7 |
| 6 x 10¹⁰ | 248.1 | 4.6 |
| 1.2 x 10¹¹ | 276.7 | 5.1 |

As can be seen the nitrocellulose containing membranes produce a larger INS than the inert PVDF membranes.

### Example 7. General method to detect proteins on nitrocellulose membranes

A Millipore^{™} GSWP04700 nitrocellulose membrane was placed on a 3 mm thick absorbent paper pad. A six mm diameter Teflon well was then placed on top of the nitrocellulose membrane. Aliquots of bovine serum albumin (BSA) solution (50 microliters) were allowed to wick through the nitrocellulose membrane into the lower adsorbent pad over a period of approximately 10 seconds. The aliquots of BSA contained 10 mg/ml, 1 mg/ml, 0.1 mg/ml, 0.01 mg/ml and 0.001 mg/ml of BSA in a phosphate buffer (PB) at pH7.4. The membranes were then washed with fifty microliters of deionized water. This procedure effectively adsorbed different quantities of a protein (BSA) onto the nitrocellulose membrane. The amount of protein adsorbed was then detected by allowing two aliquots of Au16 nanoparticles (50 microliters) to pass through the nitrocellulose membrane, followed by deionized water (50 microliters). The Au16 nanoparticles adsorb onto the protein but do not adsorb onto the nitrocellulose membrane. The membrane was dried and placed adjacent to a photoflash. The INS was then obtained as for example 5 except that each membrane was subjected to the photoflash three times with an integration time of six milliseconds for each photoflash and the INS values reported are the sum of the three INS values. Results of the summed INS values for the membrane with different quantities of adsorbed BSA are shown in table 3 below.

**TABLE 3**

| Aliquot concentration of BSA adsorbed onto nitrocellulose membrane (mg/ml) | Summed INS |
|---|---|
| 0 | 11.9 |
| 0.001 | 14.6 |
| 0.01 | 15.3 |
| 0.1 | 105.4 |
| 1 | 120.1 |
| 10 | 138.2 |

As can be seen, different concentrations of proteins can be readily discerned.

### Example 8. Enzyme sensor based on impulsive noise generation

Nitrocellulose membrane disks (6 mm diameter) were punched out of Millipore^{™} GSWP04700 nitrocellulose membranes. The disks were placed in a solution of a protein peroxidase conjugate (goat anti-rabbit peroxidase conjugate product number 0545, 2ml, diluted 500:1 ratio in PB, Sigma Chemical Company) and gently mixed for 30 minutes at room temperature. The disks were removed from the protein peroxidase conjugate solution and washed twice with PB for 3 minutes. In the present example the analyte is 3,3'-diaminobenzidine, which forms a brown coloured precipitate through the action of the enzyme. Analyte solution was prepared by diluting a stock solution of 3,3'-diaminobenzidine (0.7 mg/ml) with deionized water. The analyte stock solution also contained urea hydrogen peroxide (0.2 mg/ml). Separate disks were then placed in the solutions containing different amounts of analyte for 5 minutes. After 5 minutes the disks were then placed in water to stop further enzyme reaction. The disks were dried and placed adjacent to a photoflash. The INS was then obtained as for example 7. Results of the summed INS values for the disks with different quantities of 3,3'diaminobenzidine are shown in table 4.

**TABLE 4**

| Concentration of 3,3'-diaminobenzidine (mg/ml) | Summed INS |
|---|---|
| 0 | 15.1 |
| 0.14 | 54.5 |
| 0.28 | 105.9 |
| 0.42 | 194.3 |
| 0.56 | 221.6 |
| 0.70 | 202.6 |

### Example 9. Analyte detection and determination of analyte concentration using the impulsive noise generation based on an enzyme linked immunoassay (ELISA)

Nitrocellulose membrane disks (6 mm diameter) were punched out of Millipore^{™} GSWP04700 nitrocellulose membranes. The disks were placed in a solution of goat anti-rabbit antibody (0.1 mg/ml, 2 ml in PB, Sigma Chemical Company, product number R2004) and gently mixed for 30 minutes at room temperature. The disks were rinsed once with PB and non-specific binding sites were then blocked with casein solution (0.5% wt/vol in PB, 2 ml) for 90 minutes. As a model analyte, rabbit anti-BSA antibody (Sigma Chemical Company, product number 1520) was prepared at different concentrations in PB. Concentration ranged from 2 to 200 ng/ml. Separate disks were incubated with the rabbit anti-BSA antibody solutions for 30 minutes at room temperature. The disks were washed in PB for two minutes. The disks were then incubated with goat anti-rabbit antibody peroxidase conjugate (diluted 500:1 ratio in PB, Sigma Chemical Company product number 0545) for 10 minutes with gentle mixing. The disks were then washed in PB for three minutes. This wash was repeated twice more. Then a solution (0.7ml) of 3,3'-diaminobenzidine (0.7 mg/ml), urea hydrogen peroxide (0.2 mg/ml) in TRIS buffer (0.06M) was added to each disk and the disks were incubated for two minutes. The disks were then added to deionized water for 2 minutes. The disks were then dried and placed adjacent to a photoflash. The INS was then obtained as for example 5 except that the total integration time was six milliseconds after the photoflash was activated. Results of the INS for the disks with different quantities of the analyte (rabbit anti-BSA antibody) are shown in table 5.

**TABLE 5**

| Concentration of Rabbit anti-BSA Antibody (ng/ml) | INS |
|---|---|
| 2 | 6.7 |
| 5 | 7.3 |
| 10 | 19.6 |
| 20 | 42.2 |
| 50 | 168.2 |
| 100 | 165.4 |
| 200 | 169.4 |

### Example 10. Synthesis of Au16 gold nanoparticles conjugated with anti-rabbit IgG

The Au16-goat anti-rabbit antibody conjugate was made according to the method of Wang et al J. Virological Methods, 132 (2006) 212-215. To a solution of Au16 gold nanoparticles (2 ml) was added potassium carbonate buffer (2% wt/vol) to increase the pH of the solution to 8.6. A solution of goat anti-rabbit antibody ( ICN#55602, 30 µl, 500 µg/ml) was then added to the Au16 gold nanoparticle solution and the solution was allowed to stand for 60 minutes at room temperature to form the Au16-goat anti-rabbit antibody conjugate. The solution was then purified from excess antibody by centrifugation (14,000 rpm, 20 minutes), followed by resuspension of the Au16-goat anti-rabbit antibody conjugate pellet in PB containing 1% wt/vol of BSA.

### Example 11. Analyte detection and determination of analyte concentration using the impulsive noise generation based on adsorption of Au16/anti-rabbit IgG conjugate

Nitrocellulose membrane disks (6 mm diameter) were punched out of Millipore^{™} GSWP04700 nitrocellulose membranes. The disks were placed in a solution of goat anti-rabbit antibody (0.1 mg/ml, 2 ml in PB, Sigma Chemical Company, product number R2004) and gently mixed for 30 minutes at room temperature. The disks were rinsed once with PB and non-specific binding sites were then blocked with casein solution (0.5% wt/vol in PB, 2 ml) for 90 minutes. As a model analyte, rabbit anti-BSA antibody (Sigma Chemical Company, product number 1520) was prepared at different concentrations in PB. Concentration ranged from 2 to 200 ng/ml. Separate disks were incubated with the rabbit anti-BSA antibody solutions for 30 minutes at room temperature. The disks were washed in PB for two minutes. The disks were then incubated overnight with solutions (100 microliters) of Aul6-goat anti-rabbit antibody conjugate, made according to example 10. The disks were then washed with deionized water. The disks were then dried and placed adjacent to a photoflash. The INS was then obtained as for example 5 except that the total integration time was six milliseconds after the photoflash was activated. Results of the INS for the disks with different quantities of the analyte (rabbit anti-BSA antibody) are shown in table 5.

**TABLE 5**

| Concentration of Rabbit anti-BSA Antibody (ng/ml) | INS |
|---|---|
| 1 | 4.5 |
| 5 | 4.8 |
| 10 | 4.8 |
| 50 | 135.6 |
| 100 | 170.1 |
| 500 | 219.6 |
| 1000 | 225.7 |

### Example 12. Enhancement of assay sensitivity by autocatalytic silver deposition on the Au16 gold nanoparticles

A solution of Au16 gold nanoparticles (10 ml) was treated with cystamine hydrochloride (100 ul, 0.1M aqueous solution). Rapid cross-linking of the nanoparticles occurred as evidenced by the rapid colour change from burgundy-red to blue-black. Aliquots of this solution were diluted with water as required and were then filtered through Millipore^{™} GSWP04700 membranes. These membranes are derived from nitrocellulose and have a nominal pore size of 0.22 um. The filter area used was approximately 0.28 cm². After filtration the cross-linked nanoparticles formed a thin film on and in the pores of the membrane. The membrane segments containing the cross-linked nanoparticle films were then placed in a solution of LI silver enhancer solution from Nanoprobes Inc, NY for 15 minutes. The LI silver enhancer solution consists of a two part electroless plating solutions that autocatalytically plate silver onto gold nanoparticles, thereby increasing the size and optical density of the nanoparticles. The membrane segments were washed with deionized water. The membrane segments were then dried and placed adjacent to a photoflash. The INS was then obtained as for example 5. Results of the INS for the membrane segments that had been treated with the silver enhancer solution according to the present example are compared to the INS for membrane segments that had not been treated with the silver enhancer solution according to example 5, are shown in table 6.

**TABLE 6**

| Number of nanoparticles filtered onto membrane | INS for nitrocellulose membrane from example 5 | INS for nitrocellulose membranes enhanced with autocatalytically deposited metal onto Au16 nanoparticles |
|---|---|---|
| 6 x 10⁸ | 2.2 | 5.1 |
| 1.2 x 10⁹ | 2.4 | 5.4 |
| 2.4 x 10⁹ | 2.5 | 68.2 |
| 6 x 10⁹ | 7.0 | 127.5 |
| 1.2 x 10¹⁰ | 125.3 | 204.2 |

As can be clearly seen there is a significant increase in the INS for the nanoparticles material on the nitrocellulose membrane once treated with silver enhancer solution.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

## Claims

1. A method for measuring the presence or absence of an analyte in a sample, wherein the presence or absence of the analyte is indicated by the respective presence or absence of an impulsive noise of generated by one to more trigger elements when triggered, comprising the steps of:
(a) exposing the one or more trigger elements to a triggering impulse;
(b) detecting the impulsive noise generated by said one or more trigger elements, and wherein the trigger element interacts with a reactive substrate to generate the impulsive noise.

2. A method according to claim 1 wherein the triggering impulse is radiation.

3. A method according to claim 2 wherein the source of the radiation is derived from a photoflash.

4. A method according to claim 1, 2 or 3 wherein the reactive substrate comprises an organic polymer functionalised with organo-nitro groups.

5. A method according to any preceding claim wherein the reactive substrate contains nitrated cellulose, nitrated starch, nitrated carbohydrate such as nitro-mannitol.

6. A method according to any preceding claim wherein the reactive substrate comprises a nitrocellulose material containing a proportion of cellulose acetate.

7. A method according to any one of claims 1 to 6 wherein the trigger element is a nanoparticle.

8. A method according to any one of claims 1 to 7 wherein the trigger element comprises a gold nanoparticle of 5 to 60 nm diameter.

9. A method according to any one of claims 1 to 8, said method comprising, before steps (a) and (b), the steps of:
(i) immobilising the first receptors in a region wherein the first receptors are capable of binding to the analyte;
(ii) adding the sample to the region whereby, when analyte is present in the sample, the analyte binds to said first receptors;
(iii) adding second receptors to the region wherein the second receptors bind to the analyte and wherein the second receptors comprise said one or more trigger elements; and
(iv) removing unbound second receptors from said region.

10. A method according to any one of claims 1 to 8, said method comprising, before steps (a) and (b), the steps of:
(i) immobilising first receptors in a region wherein the first receptors are capable of binding to the analyte;
(ii) adding analyte-mimics to the region wherein the analyte-mimics bind to the first receptors and wherein the analyte-mimics comprise a trigger element that generates an impulsive noise when triggered;
(iii) adding the sample to the region whereby, when analyte is present in the sample, the analyte binds to said first receptors and displaces said analyte-mimics; and
(iv) removing unbound analyte-mimics from said region.

11. A method according to any one of claims 1 to 8, said method comprising, before steps (a) and (b), the step of:
(i) exposing said sample to an analyte-specific substrate, wherein said analyte-specific substrate generate said one or more trigger elements in the presence of analyte.

12. A method according to any one of claims 1 to 8, said method comprising, before steps (a) and (b), the steps of:
(i) exposing said sample to an analyte-specific substrate, wherein said analyte-specific substrate adsorbs or binds to said analyte and wherein said analyte-specific substrate comprises one or more trigger elements; and
(ii) removing unadsorbed or unbound analyte-specific substrate.

13. A method according to any one of claims 1 to 12, said method comprising, before steps (a) and (b), the step of:
(i) exposing said one or more trigger elements to a solution of electroless metal plating solution, wherein said electroless metal plating solution deposits metal onto the triggering elements.

## Patentansprüche

1. Verfahren zur Messung der Gegenwart oder Abwesenheit eines Analyten in einer Probe, wobei die Gegenwart oder Abwesenheit des Analyten durch die Gegenwart und/oder Abwesenheit von stoßartigem Rauschen angezeigt wird, die durch ein oder mehrere Triggerelement(e) beim Triggern erzeugt wird, umfassend der Schritte von:
(a) Aussetzen des einen Triggerelementes oder der mehreren Triggerelemente einem Triggerimpuls;
(b) Erfassen des Stoßartigen Rauschens, das durch ein oder mehrere Triggerelement(e) erzeugt wird, und wobei das Triggerelement mit einem reaktiven Substrat reagiert, um das stoßartige Rauschen zu erzeugen.

2. Verfahren nach Anspruch 1, wobei der Triggerimpuls eine Strahlung ist.

3. Verfahren nach Anspruch 2, wobei die Quelle der Strahlung von einem Blitzlicht stammt.

4. Verfahren nach Anspruch 1, 2 oder 3, wobei das reaktive Substrat ein organisches Polymer umfasst, das mit organischen Nitro-Gruppen funktionalisiert ist.

5. Verfahren nach einem der vorhergehenden Ansprüche, wobei das reaktive Substrat nitrierte Cellulose, nitrierte Stärke, nitrierte Saccharide wie etwa Nitromannit enthält.

6. Verfahren nach einem der vorhergehenden Ansprüche, wobei das reaktive Substrat ein Nitrocellulose-Material umfasst, das einen Anteil von Cellulose-Acetat enthält.

7. Verfahren nach einem der Ansprüche 1 bis 6, wobei das Triggerelement ein Nanopartikel ist.

8. Verfahren nach einem der Ansprüche 1 bis 7, wobei das Triggerlement ein Gold-Nanopartikel von 5 bis 60 nm Durchmesser umfasst.

9. Verfahren nach einem der Ansprüche 1 bis 8, wobei das Verfahren vor den Schritten (a) und (b) die Schritte umfasst:
(i) Immobilisieren der ersten Rezeptoren in einem Bereich, in dem die ersten Rezeptoren eine Bindung zu dem Analyten machen können;
(ii) Hinzufügen der Probe zu dem Bereich, wobei sich der Analyt an die ersten Rezeptoren bindet, wenn ein Analyt in der Probe vorhanden ist;
(iii) Hinzufügen zweiter Rezeptoren zu dem Bereich, wobei sich die zweiten Rezeptoren an den Analyten binden und wobei die zweiten Rezeptoren ein oder mehrere Triggerelement(e) umfassen; und
(iv) Entfernen ungebundener zweiter Rezeptoren von dem Bereich.

10. Verfahren nach einem der Ansprüche 1 bis 8, wobei das Verfahren vor den Schritten (a) und (b) die Schritte umfasst:
(i) Immobilisieren erster Rezeptoren in einem Bereich, in dem die ersten Rezeptoren eine Bindung zu dem Analyten machen können;
(ii) Hinzufügen von Analyten-Imitatoren zu dem Bereich, wobei sich die Analyten-Imitatoren an die ersten Rezeptoren binden und wobei die Analyten-Imitatoren ein Triggerelement umfassen, welches ein stoßartiges Rauschen beim Triggern erzeugt;
(iii) Hinzufügen der Probe zu dem Bereich, wobei sich der Analyt an die ersten Rezeptoren bindet und die Analyt-Imitatoren ersetzt, wenn ein Analyt gegenwärtig ist; und
(iv) Entfernen der ungebundenen Analyt-Imitatoren von dem Bereich.

11. Verfahren nach einem der Ansprüche 1 bis 8, wobei das Verfahren vor den Schritten (a) und (b) den Schritt umfasst:
(i) Immobilisieren der Probe für ein Analytspezifisches Substrat, wobei das Analytspezifische Substrat ein oder mehrere Triggerelement(e) bei der Gegenwart eines Analyten erzeugt.

12. Verfahren nach einem der Ansprüche 1 bis 8, wobei das Verfahren vor den Schritten (a) und (b) die Schritte umfasst:
(i) Immobilisieren der Probe für ein Analytspezifisches Substrat, wobei das Analytspezifische Substrat einen Analyten absorbiert oder sich an diesen bindet und wobei das Analyt spezifische Substrat ein oder mehrere Triggerelement(e) umfasst; und
(ii) Entfernen eines nicht absorbierten oder ungebundenen Analytspezifischen Substrates.

13. Verfahren nach einem der Ansprüche 1 bis 12, wobei das Verfahren vor den Schritten (a) und (b) den Schritt umfasst:
(i) Immobilisieren eines Triggerelementes oder mehrerer Triggerelemente für eine Lösung einer stromlosen Metallgalvanisierungs-Lösung, wobei die stromlose Metallgalvanisierungs-Lösung Metall auf die Triggerelemente ablagert.

## Revendications

1. Procédé de mesure de la présence ou de l'absence d'un analyte dans un échantillon, dans lequel la présence ou l'absence de l'analyte est indiquée par la présence ou l'absence respective d'un bruit impulsif généré par un ou plusieurs éléments déclencheurs, lorsqu'il(s) est (sont) déclenché(s), comprenant les étapes:
(a) d'exposition du ou des éléments déclencheurs à une impulsion de déclenchement ;
(b) de détection du bruit impulsif généré par ledit ou lesdits éléments déclencheurs, et dans lequel l'élément déclencheur interagit avec un substrat réactif pour générer le bruit impulsif.

2. Procédé selon la revendication 1, dans lequel l'impulsion de déclenchement est un rayonnement.

3. Procédé selon la revendication 2, dans lequel la source de rayonnement provient d'un flash photographique.

4. Procédé selon la revendication 1, 2 ou 3 dans lequel le substrat réactif comprend un polymère organique fonctionnalisé avec des groupes organo-nitro.

5. Procédé selon l'une quelconque des revendications précédentes, dans lequel le substrat réactif contient de la cellulose nitrée, de l'amidon nitré, de l'hydrate de carbone nitré tel que le nitro-mannitol.

6. Procédé selon l'une quelconque des revendications précédentes, dans lequel le substrat réactif comprend un matériau de nitrocellulose contenant une proportion d'acétate de cellulose.

7. Procédé selon l'une quelconque des revendications 1 à 6, dans lequel l'élément déclencheur est une nanoparticule.

8. Procédé selon l'une quelconque des revendications 1 à 7, dans lequel l'élément déclencheur comprend une nanoparticule d'or de 5 à 60 nm de diamètre.

9. Procédé selon l'une quelconque des revendications 1 à 8, ledit procédé comprenant, avant les étapes (a) et (b), les étapes:
(i) d'immobilisation de premiers récepteurs dans une région dans laquelle les premiers récepteurs sont capables de se lier à l'analyte ;
(ii) d'ajout de l'échantillon à la région, par laquelle, lorsque l'analyte est présent dans l'échantillon, l'analyte se lie auxdits premiers récepteurs ;
(iii) d'ajout de seconds récepteurs à la région dans laquelle les seconds récepteurs se lient à l'analyte et dans laquelle les seconds récepteurs comprennent ledit ou lesdits éléments déclencheurs ; et
(iv) de retrait des seconds récepteurs non liés de ladite région.

10. Procédé selon l'une quelconque des revendications 1 à 8, ledit procédé comprenant, avant les étapes (a) et (b), les étapes:
(i) d'immobilisation de premiers récepteurs dans une région dans laquelle les premiers récepteurs sont capables de se lier à l'analyte ;
(ii) d'ajout d'imitateurs d'analytes à la région dans laquelle les imitateurs d'analytes se lient aux premiers récepteurs et dans laquelle les imitateurs d'analytes comprennent un élément déclencheur qui génère un bruit impulsif lorsqu'il est déclenché ;
(iii) d'ajout de l'échantillon à la région, par laquelle, lorsque l'analyte est présent dans l'échantillon, l'analyte se lie auxdits premiers récepteurs et déplace lesdits imitateurs d'analyte ; et
(iv) de retrait des imitateurs d'analytes de ladite région.

11. Procédé selon l'une quelconque des revendications 1 à 8, ledit procédé comprenant, avant les étapes (a) et (b), l'étape :
(i) d'exposition dudit échantillon à un substrat analyte-spécifique, dans lequel ledit substrat analyte-spécifique génère ledit ou lesdits éléments déclencheurs en présence d'analyte.

12. Procédé selon l'une quelconque des revendications 1 à 8, ledit procédé comprenant, avant les étapes (a) et (b), les étapes:
(i) d'exposition dudit échantillon à un substrat analyte-spécifique, dans lequel ledit substrat analyte-spécifique adsorbe ledit analyte ou se lie à celui-ci et dans lequel ledit substrat analyte-spécifique comprend un ou plusieurs éléments déclencheurs ; et
(ii) de retrait du substrat analyte-spécifique non adsorbé ou non lié.

13. Procédé selon l'une quelconque des revendications 1 à 12, ledit procédé comprenant, avant les étapes (a) et (b), l'étape:
(i) d'exposition dudit ou desdits éléments déclencheurs à une solution de solution de placage métallique anélectrolytique, ladite solution de placage métallique anélectrolytique déposant du métal sur les éléments de déclenchement.
